# FASCICULE DE BREVET EUROPEEN

(11) **EP 1 433 449 B1**
(45) Date de publication et mention de la délivrance du brevet: **07.03.2012**
(21) Numéro de dépôt: 03292453.2
(22) Date de dépôt: 03.10.2003
(51) Int. Cl.: A61F 11/08

(54) **Dispositif auditif et son procédé de réalisation**
Hörgerät und Verfahren zur seiner Herstellung
Hearing device and method for producing the same

(30) Priorité: 04.10.2002 FR 0212376; 04.10.2002 FR 0212377; 10.07.2003 FR 0308485
(43) Date de publication de la demande: 30.06.2004
(73) Titulaire: Auditech Innovations, 76160 Darnetal (FR)
(72) Inventeur: Roussel, Pascal, 76160 Saint-Martin-du Vivier (FR)
(74) Mandataire: Novagraaf Technologies

(56) Documents cités:
- EP-A- 1 071 307
- FR-A- 2 667 500
- US-A- 3 131 241
- US-A- 3 833 701
- US-A- 5 718 244

## Description

La présente invention se rapporte au domaine des dispositifs auditifs et leurs procédés de réalisation/fabrication. La présente invention se rapporte plus particulièrement à la réalisation par l'utilisateur lui-même d'une matrice d'oreille en vu de la fabrication simplifiée d'embouts d'oreille destinés à un dispositif auditif de protection contre le bruit.

À l'heure actuelle, on connaît différents systèmes de protections individuelles contre le bruit et notamment les casques, les bouchons en mousse, les embouts moulés équipés d'évents ou de perçages. Les systèmes de protection auditive utilisant le principe de moulage du conduit auditif, dans le but de protéger les personnels exposés aux bruits, remontent seulement à une quinzaine d'années durant cette période, les demandes d'améliorations acoustiques ou ergonomiques faites par les utilisateurs ont été sans cesse plus nombreuses, sans que le marché propose de produit en adéquation avec ces demandes.
Ces embouts auriculaires sont réalisés d'après un modèle obtenu par injection, jusque dans le conduit auditif interne ou conduit osseux, d'une pâte silicone molle comme décrit dans le EP 1 071 307. Cette injection est faite par un spécialiste à l'aide d'une seringue. La réalisation de ce modèle, appelé aussi empreinte, dans l'état actuel de la technique est parfaitement adaptée pour les équipements relevant d'une aide auditive, mais freine considérablement, par sa lourdeur de mise en place, la diffusion de protections antibruit de type moulé sur mesure dans le domaine de l'équipement de sécurité des salariés de l'industrie.
La fabrication de l'embout moulé d'oreille, pour la protection antibruit, est actuellement le dérivé d'un procédé pour obtenir un embout moulé utilisé avec l'adjonction d'une aide auditive. Ce procédé de fabrication, adapté pour répondre aux besoins de santé, est, par addition d'un certain nombre d'étapes visant à le transformer, proposé comme antibruit dans l'industrie. Le temps de fabrication et les coûts de production n'en sont que plus élevés.
Ainsi, l'art antérieur connaît notamment la demande de brevet WO 9734443 qui décrit une prothèse auditive comprenant un ensemble électronique fixé dans un boîtier cylindrique, ce dernier étant fixé dans une ouverture ménagée dans un moulage de l'oreille. L'ensemble électronique comprend une bande à circuits imprimés sur laquelle sont placés un haut-parleur, un microphone et des composants électroniques formant un circuit de traitement des signaux pour amplifier les sons. Le moulage de l'oreille est fabriqué en un matériau doux, résistant et souple de telle sorte qu'il s'insère étroitement dans l'oreille de l'utilisateur. La prothèse auditive présente une conception comportant un nombre minime de composants et peut être facilement assemblée automatiquement. L'on obtient ainsi une prothèse auditive qui est relativement assez peu onéreuse pour pouvoir être jetable.

Pour obtenir des atténuations importantes, dans l'état actuel de la technique, les diamètres de perçage utilisés sont extrêmement faibles et rapidement bouchés à l'utilisation, il en découle une forte variation de l'atténuation attendue et une autophonie accrue (sensation d'oreille bouchée.)
Dans l'état actuel de la technique, les poignées de préhension des protecteurs sont réalisées dans la même matière que l'embout et après plusieurs temps d'utilisation peuvent se fissurer et se déchirer. Les systèmes d'attache de cordelette de liaison percés dans la matière de l'embout le fragilisent et après un certain temps d'utilisation elles se déchirent. L'insertion à la demande, pour le secteur de l'agroalimentaire, d'une bille métallique dans l'embout moulé détectable par variation de champs magnétiques, entraîne un travail supplémentaire sur l'embout et un temps de fabrication augmenté.
Par conception, les embouts auriculaires ont des faibles dimensions et par manque de place ne permettent pas d'y noter des informations importantes telles que le nom de l'utilisateur, la date de mise en service, les dates de révision. La différentiation droite et gauche, quand elle existe, est réalisée par un point coloré sur l'un des embouts. Ce point, réalisé en surface sur l'embout, est petit et parfois peu visible, il s'altère et s'estompe dans le temps, il entraîne un temps de fabrication augmenté.

La présente invention entend remédier aux inconvénients de l'art antérieur en proposant un procédé de réalisation d'embouts moulés d'oreille simple et peu onéreux permettant aux destinataires de réaliser eux-mêmes une matrice de chacune de leurs oreilles ainsi que le contre-moule utilisé pour fabriquer in fine chacun des embouts moulés d'oreille. Par ailleurs, lors de la fabrication desdits embouts, le procédé selon l'invention propose l'insertion d'une étiquette sans contact, par exemple du type RFID, permettant d'inscrire un grand nombre d'information et de procéder au suivi du dispositif auditif de protection contre le bruit.

Pour ce faire, la présente invention concerne un procédé de réalisation d'un dispositif auditif, destiné à être adapté à l'oreille d'un utilisateur pour y être inséré, comportant une étape de réalisation d'une matrice d'oreille grâce à un élastomère durcissant pour prendre la forme de l'oreille de l'utilisateur et une étape de fabrication d'un embout moulé d'oreille par contre-moulage à partir de ladite matrice, caractérisé en ce que l'étape de réalisation de la matrice d'oreille comprend les étapes suivantes :
- dépôt d'une quantité suffisante d'élastomère, par exemple du silicone, sur un support disposant d'un réservoir contenant au moins en partie le susdit élastomère,
- pression sur un piston servant à éjecter l'élastomère présent dans le susdit réservoir et pousser l'élastomère dans l'oreille de l'utilisateur.

Avantageusement, grâce au procédé selon l'invention, l'utilisateur pourra réaliser lui-même la matrice d'oreille.

Avantageusement, le procédé de l'invention comprendra, à la suite de l'étape de réalisation de la matrice d'oreille, une étape d'ébavurage de la matrice.

Avantageusement, le procédé comprendra en outre une étape de fixation de la matrice d'oreille sur une plaque puis la fixation sur ladite plaque d'un contenant délimitant un volume dans lequel loge la matrice d'oreille.

De préférence, le contenant sera rempli d'un matériau semi-liquide à mémoire de forme, tels que par exemple un gel hydrocoloïde ; ledit matériau constituant le contre-moule nécessaire pour la fabrication de l'embout moulé d'oreille.

De préférence, l'étape de fabrication de l'embout moulé d'oreille comprendra une étape d'insertion d'une étiquette sans contact pour la lecture/écriture d'information, tels que du type RFID, dans le contre-moule.

La présente invention se rapporte également à un dispositif pour la mise en oeuvre du procédé décrit ci-dessus. Ce dispositif comprend un support relativement plat disposant d'un réservoir s'étendant en retrait du plan dudit support ainsi que d'un piston mobile dans ledit réservoir.

Avantageusement, le susdit réservoir consistera en un tube cylindrique.

L'invention porte également sur un dispositif de protection auditive individuel, caractérisé en ce qu'il comprend un embout moulé d'oreille pour chacune des oreilles de l'utilisateur ; lesdits embouts moulés d'oreille étant réalisé et fabriqué par le procédé décrit précédemment.

De préférence, les embouts moulés d'oreille comprendront au moins une étiquette sans contact pour la lecture/écriture d'information, telle que du type RFID.

Avantageusement, le dispositif de l'invention comprendra un ensemble poignée-rétention, chacun des embouts moulés d'oreille étant fixé à l'élément de rétention.

Avantageusement, l'élément poignée pourra comprendre un logement pour loger une pièce métallique pour assurer sa détection par exemple par variation d'un champ magnétique.

De préférence, l'élément rétention comprend une partie coulissant dans une partie de forme complémentaire de l'élément poignée ; les deux parties présentant par exemple des formes cylindriques.

Avantageusement, la poignée comprendra au moins deux membranes, située dans sa partie en vis-à-vis de la partie complémentaire coulissante de l'élément rétention, pour former des surfaces en regard. Les membranes sont utilisées pour rendre un effet vibratoire et parfois poreux. Ainsi, les deux membranes en regard seront distantes d'une longueur caractérisant le passage de ladite fréquence. À titre d'exemple, pour 1 KHz, la distante L entre les deux membranes pourra être égale à 1 mm (millimètre).

Avantageusement, le volume entre les deux membranes sera compris entre 0,14 cm³ et 0,006 cm³.

Selon une possibilité offerte par l'invention, les deux embouts moulés pourront être reliés par un cordon, par exemple fixé à l'élément poignée.

Selon une autre possibilité offerte par l'invention, les deux embouts moulés d'oreille seront reliés par un arceau souple dont la fixation respective, par exemple assurée par vissage sur chaque élément poignée, aux embouts moulés est réglable de manière à leur conférer une pression variable F.

De préférence, chaque embout moulé d'oreille comportera un signe distinctif, tels que par exemple une couleur différente, permettant d'identifier l'embout de l'oreille gauche et celui de l'oreille droite.

Avantageusement, l'ensemble poignée-rétention sera en matière plastique ou en tout autre matière différente de celle des embouts moulés d'oreille.

on comprendra mieux l'invention à l'aide de la description, faite ci-après à titre purement explicatif, d'un mode de réalisation de l'invention, en référence aux figures annexées :
- la figure 1 illustre une vue en coupe du dispositif selon l'invention pour la réalisation de la matrice d'oreille par l'utilisateur lui-même grâce à un matériau élastomère ;
- la figure 2 illustre une vue schématique des doigts d'un utilisateur sur le point d'appliquer sur l'une de ses oreilles le dispositif de réalisation avec le matériau élastomère représenté sur la figure 1 ;
- la figure 3 illustre une vue en coupe de la plaque sur laquelle est fixée la matrice d'oreille ainsi que le contenant pour l'injection d'un matériau à mémoire de forme ;
- la figure 4 illustre une vue en coupe du contre-moule représenté sur la figure 3 dans lequel on a placé une étiquette sans contact ;
- la figure 5 illustre une vue en coupe d'un embout moulé d'oreille disposant d'une forme adaptée pour être fixé à l'élément rétention ainsi que l'ensemble poignée-rétention relié à un cordon ;
- la figure 6 une vue de face de l'élément poignée comportant deux orifices circulaires, l'un pour disposer l'extrémité du cordon et l'autre pour loger une pièce métallique ;
- la figure 7 illustre une vue en coupe de l'élément rétention fixé à un embout moulé d'oreille ainsi qu'à la poignée, elle-même reliée à un cordon ;
- la figure 8 illustre une vue en coupe d'un mode de réalisation d'un dispositif auditif ou acoustique pour la protection contre le bruit dans lequel les deux embouts moulés d'oreille sont reliés par un arceau souple ;
- les figures 9 et 10 illustrent une vue en coupe de la partie de fixation entre l'arceau et la poignée montrant en particulier le degré de liberté de l'embout en fonction du serrage de la vis de fixation sur la poignée.

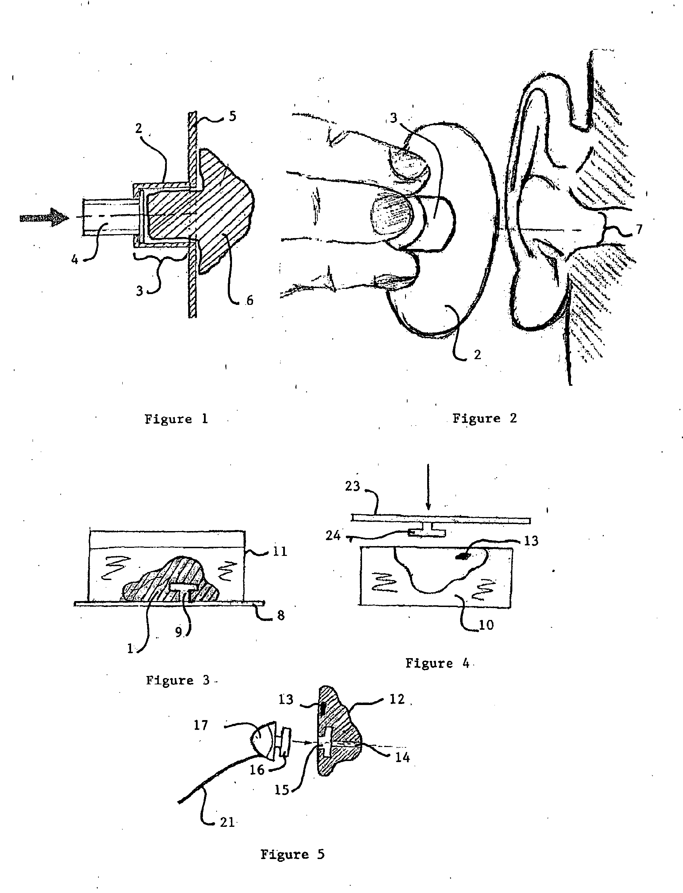

Selon l'invention, illustré figure 1, le dispositif pour réaliser la matrice d'oreille 1 soi-même se compose d'un simple support 2, par exemple de forme ovoïdale comme représenté sur la figure 2, comportant en son centre un retrait faisant office de réservoir 3. Ce retrait 3, ou réservoir, est ici de forme cylindrique, mais on pourra prévoir toutes sortes d'autres formes telles que par exemple rectangulaire ou carrée. Ce retrait 3 est muni d'un simple piston 4 coulissant apte à coulisser depuis l'extrémité de la paroi intérieure dudit retrait 3 jusqu'à sa limite avec le plan de la surface plane 5 du support 2. On pourra bien sûr envisager que ce piston 4 soit monté avec des ressorts pour aider l'utilisateur lors de la pression du piston, mais le piston 4 représenté dans l'exemple choisi pour illustrer l'invention ne comprend aucun ressort. Le dispositif de l'invention, consistant en ce support 2, présente donc une coupe de section sensiblement en forme de T et la forme ovoïdale ou sensiblement sphérique du plan du support est choisie pour présenter la surface de contact avec l'oreille la plus grande possible de sorte que l'utilisateur peut exercer une pression relativement importante sur le support 2 et le piston 4 sans endommager son oreille. La partie du retrait 3 doit être présentée sensiblement en face du conduit de l'oreille de manière à ce que, lors de l'application d'une pression, l'élastomère 6 s'enfonce au mieux dans le conduit auditif 7.

Une fois la matrice 1 réalisée, c'est-à-dire suite à l'introduction de l'élastomère 6 dans l'oreille 7 de l'utilisateur et le durcissement consécutif dudit élastomère, l'utilisateur retire l'élastomère 6 dorénavant présent sous une forme semi-solide et le place sur une plaque 8. Cette plaque 8 dispose, sur l'une des faces sur laquelle on vient placer la matrice 1, d'une colle ou de tout autre moyen de fixation apte à fixer solidement le matériau élastomère de la matrice d'oreille 1. Par ailleurs, comme cela est visible sur la figure 3 ou 4, on pourra prévoir que la plaque 8 comporte une protubérance 9. Cette protubérance en forme de pic 9 sert essentiellement à maintenir et à fixer la matrice d'oreille 1 pour la retirer dès que le contre-moule 10 a été réalisé. Ainsi, une fois la matrice d'oreille 1 fixée à la plaque 8, on place un contenant 11, par exemple de forme parallélépipédique, de manière à englober ou contenir ladite matrice 1. Ce contenant 11 possède au moins une ouverture par laquelle on introduit un semi-liquide à mémoire de forme, tel que par exemple un gel hydrocoloïde, apte à prendre forme solide après un certain temps, par exemple après un refroidissement. De cette manière, on réalise un contre-moule 10 rapidement et sans difficulté, et on n'abîme pas le contre-moule 10 réalisé en raison des caractéristiques d'élasticité de l'élastomère formant la matrice 1. Bien entendu, tout autre matériau adéquat pour former un solide, après adaptation aux dimensions et à la forme de la matrice d'oreille 1, pourra être envisagé à la place du semi-liquide choisi pour illustrer la présente invention.

On retire la matrice d'oreille 1 pour disposer du contre-moule 10. on utilisera pour réaliser les embouts moulés d'oreille 12 un plastique ou un élastomère disposant de caractéristiques mécaniques adéquats pour résister à l'usure et présenter une rigidité mais également une certaine souplesse nécessaire pour son introduction et son maintien dans le conduit auditif 7. Il est important de noter qu' à ce stade, il est placé au moins une étiquette sans contact 13 contre les parois du contre-moule 10, de préférence à proximité de l'extrémité supérieure du contre-moule 10, de sorte que l'étiquette 13, par exemple de type RFID, soit facilement accessible et à faible distance d'un lecteur/enregistreur d'information de ladite étiquette sans contact, non représenté sur les figures annexées.

Grâce à l'étiquette sans contact 13, le fabricant pourra, à l'aide d'un système d'écriture/lecture de puce, ou étiquette sans contact, noter sur la puce un certain nombre de renseignements, comme par exemple : la référence et le type du produit, la date de fabrication, le numéro de série, le nom ou la référence du distributeur, le nom ou la référence de l'utilisateur, la ou les dates de péremption ou de révision. Ces informations primaires pourront par ailleurs être protégées en écriture et certaines informations du fabricant pourront être protégées à la lecture par un code d'accès. Le distributeur, quant à lui, pourra noter à l'aide d'un système informatique ou d'un appareil portable permettant la lecture des informations du fabricant sur la puce électronique, ou étiquette sans contact, et la lecture/écriture de ses propres données, pourra ainsi noter via le pointeur un certain nombre de renseignement sur l'embout moulé, comme la date de livraison, le nom de l'utilisateur, différents renseignements commerciaux etc.... De la même manière que précédemment, certaines informations du distributeur pourront être protégées à la lecture par un code d'accès. On peut également envisager que l'utilisateur final puisse utiliser, de la même manière qu'énoncé ci-dessus, la puce électronique ou étiquette sans contact.

Une station centrale pourra également être envisagée dans laquelle sera stocké l'ensemble des données relatives aux différentes puces électroniques ou étiquettes sans contact implantées dans les embouts moulés d'oreille 12.

Ensuite, l'embout moulé d'oreille 12 est usiné ou travaillé de manière à réaliser un orifice 14, dont on a au préalable déterminé au préalable les dimensions en vu de l'atténuation désirée des sons extérieurs. Par ailleurs, on réalise un logement 15, par exemple en forme de T, pour loger l'élément rétention 16 qui lui-même présente une forme en T de même taille et dimensions. On pourra prévoir de disposer sur la face externe de l'élément rétention 16 une colle, afin de fixer encore plus solidement ledit élément rétention 16 et l'embout moulé d'oreille 12.

Comme cela est visible sur les figures, l'élément poignée 17 est fixé à l'élément rétention 16, par exemple grâce à une butée autorisant le coulissement d'une partie cylindrique 18 de l'élément rétention 16 dans une partie complémentaire de l'élément poignée 17 sans permettre la séparation des deux éléments 16 et 17. Par ailleurs, la poignée 17 comprend deux orifices ou logements 19 et 20 situés diamétralement opposés sur la face sensiblement circulaire de la poignée 17, l'un 20 des deux logements servant à loger l'extrémité du cordon 21 reliant, dans ce mode de réalisation, les deux embouts 12 à disposer dans les deux oreilles, et l'autre logement 19 servant à loger une pièce métallique 22 pour la détection du dispositif lors de son passage dans un champ magnétique.

Le support 2 pourra être transparent ou opaque selon les choix particuliers de l'utilisateur. On peut envisager que la matrice d'oreille 1 se présente initialement comme une boule de 1,5 cm de diamètre, cette quantité de matière silicone ou analogue devant être suffisante pour réussir une empreinte totale du conduit auditif 7, soit la conque, le crochet ou Cymba de conque et l'orifice du conduit auditif.

Autrement dit précédemment, la matrice d'oreille 1, après ébavurage, sera positionnée avec le pic 9 sur une plaque de fond 8, un contenant 11 est ensuite positionné autour de la matrice 1 de sorte à former un récipient.
Le récipient ainsi formé sera rempli de gel hydrocoloïde liquide à 45° Celsius. On attendra le complet refroidissement de celui-ci et son état de mémoire de forme pour démouler l'ensemble. Le gel comme la matrice ayant des propriétés élastiques, le démoulage se fera sans déchirement des matières. on obtiendra alors un contre-moulage 10 dans lequel on versera un silicone liquide ayant les propriétés sanitaires et mécaniques voulues après réticulation. On prendra soin, avant réticulation du silicone, de positionner près de la surface la puce électronique de traçabilité 13, ou étiquette sans contact, et une plaque 23 avec sa partie de rétention 24.
Pour des raisons de rapidité de production et pour la suppression de possibles micro-bulles dans la matière, la réticulation du silicone pourra se faire sous une pression de 3 à 4 bars avec une température de 40 à 60° dans un autoclave. À cet effet, la plaque 23 comprendra un perçage d'équilibrage des pressions.
Après réticulation du silicone, le démoulage du contre-moule 10 et de la plaque 23 avec sa rétention 24 se fera sans déchirement du fait des propriétés d'élasticité du silicone.
L'embout auriculaire 12 ainsi réalisé avec sa partie évidée permettra l'insertion et le maintien mécanique de l'élément de rétention 16.

La partie, appelée rétention 16, est utilisée de la façon suivante : par l'intermédiaire de sa partie cylindrique 18, la partie 18 vient s'engager dans la partie complémentaire de la poignée 17. Cet engagement est limité dans sa course au maximum de sa longueur par la butée constituée par le fond de la partie cylindrique de la poignée 17. Le diamètre de la partie cylindrique 18 de l'élément rétention 16 se bloquant dans la partie complémentaire de l'élément poignée 17sera compris entre 4mm et 6mm.
La longueur de l'élément rétention 16 est fonction de la dureté shore de la matière choisie pour réaliser l'embout moulé d'oreille 12, en l'occurrence du silicone, et variera de 1 à 5 mm.
Dans l'exemple choisi pour illustrer l'invention, à la liaison des parties poignée 17 et rétention 16, et avant assemblage, vient se positionner une bulle d'air 25 sans pression comprise dans une enveloppe souple, cette bulle 25 pourra être remplie de liquide ou de matériaux micro-bullés étanches, toujours comprise dans une enveloppe souple.
Lors de l'assemblage des parties poignée 17 et rétention 16, la partie cylindrique 18 de l'élément rétention 16 venant en butée, la bulle 25 est comprimée et forme des membranes, non représentées sur les figures annexées.
Pour une onde sonore arrivant sur la première surface, seulement une partie de cette onde passera au travers de la deuxième surface de la bulle 25 en regard et arrivera vers le conduit auditif 7 par le perçage 14 pratiqué dans l'élément rétention 16 et l'embout moulé d'oreille 12.
Pour réguler l'onde sonore à la valeur voulue, on modifiera les éléments suivants :
- la longueur et donc la pression exercée sur la bulle 25, les surfaces varieront et laisseront passer plus ou moins d'ondes sonores,
- la variation des surfaces et des perçages 14 y amenant pratiqués dans les différents éléments 12 et 16, qui seront compris entre 4,5 mm² et 12 mm²,
- la distance entre les deux surfaces en regard qui sera comprise entre 0,5 et 5 mm. La bulle 25 comprimée aura donc un volume compris entre 0.14 cm³ et 0.006 cm³.

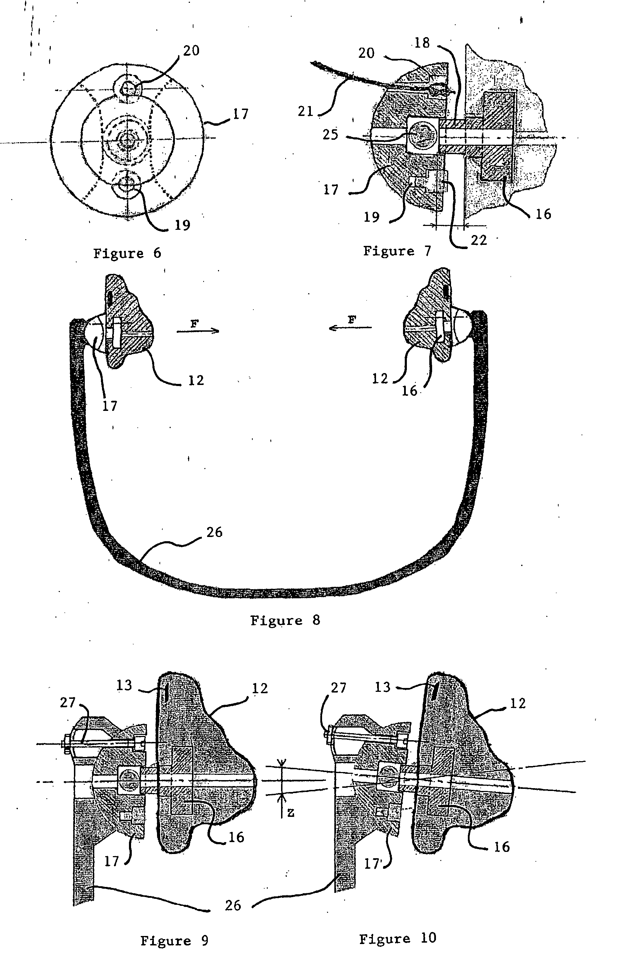

Comme cela est visible sur la figure 8, selon un autre mode de réalisation du dispositif acoustique de protection contre le bruit, on pourra prévoir de relier les deux embouts moulés d'oreille 12 avec un arceau 26 relativement souple dont les extrémités sont respectivement fixées, ici par vissage aux deux poignées 17. Ainsi, comme cela est visible sur les figures 9 et 10, en vissant fortement la vis 27 entre l'extrémité de l'arceau 26 et la poignée 17, cette dernière 17 dispose d'une très faible liberté de rotation, tandis que dans le cas où la vis 27 est faiblement fixée, ou avec lâcheté, la poignée 17 dispose d'un degré de liberté en rotation plus important. L'avantage de l'arceau 26 par rapport à la solution avec le cordon 21 réside dans le fait que l'arceau 26 agit en pression selon une force F, représentée sur la figure 8, respectivement sur les deux embouts moulés d'oreille 12 de manière à pousser lesdits embouts 12 respectivement dans le conduit 7 de chaque oreille de l'utilisateur. Les figures 9 et 10 montrent la variation d'inclinaison possible Z permettant de régler le bon positionnement d'angle de l'embout sur les oreilles.

Comme nous venons de le voir, le cordon souple 21, qui relit la paire d'antibruit 12, ou embout moulé d'oreille, pourra être remplacé par un arceau 26 d'un rayon et d'une forme permettant son positionnement sous la mâchoire ou sur la nuque de l'utilisateur. L'arceau 26 exercera une pression dans le sens des flèches F visant à appuyer les embouts moulés d'oreilles 12 spécifiques assemblés à l'ensemble poignée-rétention contre les oreilles.
Dans l'exemple choisi pour illustrer l'invention, l'arceau 26 sera fixé à chacun des antibruits 12 par une vis plastique 27 passant par un perçage réalisé dans chaque poignée 17. Le blocage par serrage de l'écrou de fixation 27, ou vis, ne se fera qu'après avoir pré-positionné les antibruits 12 et vérifié leurs bons appuis sur les oreilles.

L'invention est décrite dans ce qui précède à titre d'exemple. Il est entendu que l'homme du métier est à même de réaliser différentes variantes de l'invention sans pour autant sortir du cadre du brevet.

## Revendications

1. Procédé de réalisation d'un dispositif auditif, destiné à être adapté à l'oreille d'un utilisateur pour y être inséré, comportant une étape de réalisation d'une matrice d'oreille (1) grâce à un élastomère (6) durcissant pour prendre la forme de l'oreille (7) de l'utilisateur et une étape de fabrication d'un embout moulé d'oreille (12) par contre-moulage à partir de ladite matrice (1), **caractérise en ce que** l'étape de réalisation de la matrice d'oreille (1) comprend les étapes suivantes :
- dépôt d'une quantité suffisante d'élastomère (6), par exemple du silicone, sur un support (2) disposant d'un réservoir (3) contenant au moins en partie le susdit élastomère (6),
- pression sur un piston (4) servant à éjecter l'élastomère (6) présent dans le susdit réservoir (3) et pousser l'élastomère (6) dans l'oreille (7) de l'utilisateur.

2. Procédé de réalisation d'un dispositif' auditif selon la revendication 1, **caractérisé en ce que** l'utilisateur réalise lui-même la matrice d'oreille (1).

3. Procédé de réalisation d'un dispositif auditif selon la revendication 1 ou la revendication 2, **caractérisé en ce qu'**il comprend, à la suite de l'étape de réalisation de la matrice d'oreille (1), une étape d'ébavurage de la matrice (1).

4. Procédé de réalisation d'un dispositif auditif selon l'une quelconque des revendications précédentes, **caractérisé en ce qu'**il comprend en outre une étape de fixation de la matrice d'oreille (1) sur une plaque (8) puis la fixation sur ladite plaque (8) d'un contenant (11) délimitant un volume dans lequel loge la matrice d'oreille (1)

5. Procédé de réalisation d'un dispositif auditif selon la revendication 4, **caractérisé en ce que** le contenant (11) est rempli d'un matériau semi-liquide à mémoire de forme, tels que par exemple un gel hydrocoloïde ; ledit matériau constituant le contre-moule (10) nécessaire pour la fabrication de l'embout moulé d'oreille (12).

6. Procédé de réalisation d'un dispositif auditif selon l'une quelconque des revendications précédentes, **caractérisé en ce que** l'étape de fabrication de l'embout moulé d'oreille (12) comprend une étape d'insertion d'une étiquette sans contact (13) pour la lecture/écriture d'information, tels que du type RFID, dans le contre-moule (10).

7. Dispositif pour la mise en oeuvre du procédé selon l'une quelconque des revendications 1 à 6, **caractérisé en ce qu'**il comprend un support (2) relativement plat disposant d'un réservoir (3) s'étendant en retrait du plan (5) dudit support (2) ainsi que d'un piston mobile (4) dans ledit réservoir (3).

8. Dispositif selon la revendication 7, **caractérisé en ce que** le réservoir (3) consiste en un tube cylindrique.

9. Dispositif de protection auditive individuel, **caractérisé en ce qu'**il comprend un embout moulé d'oreille (12) pour chacune des oreilles de l'utilisateur, lesdits embouts moulés d'oreille (12) étant réalisé et fabriqué par le procédé selon les revendications 1 à 6.

10. Dispositif selon la revendication 9, **caractérisé en ce que** les embouts moulés d'oreille (12) comprennent au moins une étiquette sans contact (13) pour la lecture/écriture d'information, tels que du type RFID.

11. Dispositif selon l'une quelconque des revendications 9 à 10, **caractérisé en ce qu'**il comprend un ensemble poignée-rétention, chacun des embouts moulés d'oreille (12) étant fixé à l'élément de rétention (16).

12. Dispositif selon la revendication 11, **caractérisé en ce que** l'élément poignée (17) comprend un logement (19) pour loger une pièce métallique (22) pour assurer sa détection par exemple par variation d'un champ magnétique.

13. Dispositif selon la revendication 11, **caractérisé en ce que** l'élément rétention (16) comprend une partie coulissant (18) dans une partie de forme complémentaire de l'élément poignée (17) ; les deux parties présentant par exemple des formes cylindriques.

14. Dispositif selon la revendication 13, **caractérisé en ce que** la poignée (17) comprend au moins deux membranes, situées dans sa partie en vis-à-vis de la partie complémentaire coulissante (18) de l'élément rétention (16), pour former des surfaces en regard.

15. Dispositif selon la revendication 11, **caractérisé en ce que** les deux embouts moulé d'oreille (12) sont reliés par un cordon (21), par exemple fixé à l'élément poignée (17).

16. Dispositif selon la revendication 11, **caractérisé en ce que** les deux embouts moulé d'oreille (12) sont reliés par un arceau souple (26) dont la fixation respective, par exemple assurée par vissage sur chaque élément poignée (17), aux embouts moulés (12) est réglable de manière à leur conférer une pression variable F.

17. Dispositif selon l'une quelconque des revendications 9 à 16, **caractérisé en ce que** chaque embout moulé d'oreille (12) comporte un signe distinctif, tels que par exemple une couleur différente, permettant d'identifier l'embout de l'oreille (12) gauche et celui de l'oreille droite.

18. Dispositif selon la revendication 11, **caractérisé en ce que** l'ensemble poignée-rétention est en matière plastique ou en tout autre matière différente de celle des embouts moulés d'oreille (12).

19. Dispositif selon la revendication 14, **caractérisé en ce que** le volume entre les deux membranes est compris entre 0,14 cm³ et 0,006 cm³.

## Claims

1. Method for producing a hearing device, intended to be fitted to a user's ear for insertion therein, comprising a step for producing an ear matrix (1) by means of an elastomer (6) setting to adopt the shape of the user's ear (7) and a step for manufacturing a moulded ear tip (12) by means of counter moulding using said matrix (1), **characterised in that** the step for producing the ear matrix (1) comprises the following steps:
- depositing a sufficient quantity of elastomer (6), for example silicone, on a supporting base (2) having a receptacle (3) at least partially containing said elastomer (6),
- pressing on a plunger (4) used to eject the elastomer (6) contained in said receptacle (3) and push the elastomer (6) into the user's ear (7).

2. Method for producing a hearing device according to claim 1, **characterised in that** the user produces the ear matrix (1) him/herself.

3. Method for producing a hearing device according to claim 1 or claim 2, **characterised in that** it comprises, following the step for producing the ear matrix (1), a step for trimming the matrix (1).

4. Method for producing a hearing device according to any of the above claims, **characterised in that** it further comprises a step for mounting the ear matrix (1) on a plate (8) followed by the mounting on said plate (8) of a container (11) defining a volume wherein the ear matrix (1) is housed.

5. Method for producing a hearing device according to claim 4, **characterised in that** the container (11) is filled with a semi-liquid shape-memory material, such as for example a hydrocolloid gel; said material forming the counter-mould (10) required to manufacture the moulded ear tip (12).

6. Method for producing a hearing device according to any of the above claims, **characterised in that** the step for manufacturing the moulded ear tip (12) comprises a step for inserting a contactless label (13) for reading/writing information, such as the RFID type, in the counter-mould (10).

7. Device for implementing the method according to any of claims 1 to 6, **characterised in that** it comprises a relatively flat supporting base (2) having a receptacle (3) recessed from the plane (5) of said supporting base (2) and a movable plunger (4) in said receptacle (3).

8. Device according to claim 7, **characterised in that** the receptacle (3) consists of a cylindrical tube.

9. Personal hearing protection device, **characterised in that** it comprises a moulded ear tip (12) for each of the user's ears, said moulded ear tips (12) being produced and manufactured by means of the method according to claims 1 to 6.

10. Device according to claim 9, **characterised in that** the moulded ear tips (12) comprise at least one contactless label (13) for reading/writing information, such as the RFID type.

11. Device according to any of claims 9 to 10, **characterised in that** it comprises a handle-holding assembly, each of the moulded ear tips (12) being mounted on the holding element (16).

12. Device according to claim 11, **characterised in that** the handle element (17) comprises a housing (19) for housing a metal part (22) for the detection thereof for example by varying a magnetic field.

13. Device according to claim 11, **characterised in that** the holding element (16) comprises a sliding portion (18) in a complementary shaped portion of the handle element (17); both portions having cylindrical shapes, for example.

14. Device according to claim 13, **characterised in that** the handle (17) comprises at least two membranes, situated in the portion thereof opposite the complementary sliding portion (18) of the holding element (16), to form opposite surfaces.

15. Device according to claim 11, **characterised in that** the two moulded ear tips (12) are connected by a cord (21), for example attached to the handle element (17).

16. Device according to claim 11, **characterised in that** the two moulded ear tips (12) are connected by a flexible arch (26), the respective mounting whereof, for example, by means of screwing on each handle element (17),on the moulded tips (12) is adjustable so as to provide same with a variable pressure F.

17. Device according to any of claim 9 to 16, **characterised in that** each moulded ear tip (12) comprises a distinctive sign, such as for example a different colour, for identifying the tip of the left ear (12) and that of the right ear.

18. Device according to claim 11, **characterised in that** the handle-holding assembly is made of plastic or any other material different to that of the moulded ear tips (12).

19. Device according to claim 14, **characterised in that** the volume between the two membranes is between 0.14 cm³ and 0.006 cm³.

## Patentansprüche

1. Verfahren zur Realisierung einer Hörvorrichtung, die dazu vorgesehen ist, an das Ohr eines Benutzers angepasst zu werden, um in das Ohr gesteckt zu werden, umfassend einen Schritt zur Realisierung einer Ohrmatrix (1) mit Hilfe eines härtenden Elastomers (6), um die Form des Ohrs (7) vom Benutzer anzunehmen und einen Schritt der Herstellung eines Ohrpassstücks (12) durch Gegen-Formpressen anhand der Matrix (1), **dadurch gekennzeichnet, dass** der Schritt zur Realisierung der Ohrmatrix (1) die folgenden Schritte umfasst:
- Auftragen einer ausreichenden Menge von Elastomer (6), beispielsweise Silikon, auf einen Träger (2), der über einen Vorratsbehälter (3), der zumindest teilweise das vorgenannte Elastomer (6) enthält, verfügt,
- Druck auf einen Kolben (4), um das Elastomer (6), das im vorgenannten Vorratsbehälter (3) vorhanden ist, auszutreiben, und um das Elastomer (6) in das Ohr (7) des Benutzers zu drücken.

2. Verfahren zur Realisierung einer Hörvorrichtung nach Anspruch 1, **dadurch gekennzeichnet, dass** der Benutzer selbst die Ohrmatrix (1) realisiert.

3. Verfahren zur Realisierung einer Hörvorrichtung nach Anspruch 1 oder nach Anspruch 2, **dadurch gekennzeichnet, dass** es im Anschluss an den Schritt zur Realisierung der Ohrmatrix (1) einen Schritt zum Entgraten der Matrix (1) umfasst.

4. Verfahren zur Realisierung einer Hörvorrichtung nach einem der vorhergehenden Ansprüche, **dadurch gekennzeichnet, dass** es des Weiteren einen Schritt zur Befestigung der Ohrmatrix (1) auf einer Platte (8) umfasst, und anschließend die Befestigung auf der vorgenannten Platte (8) von einem Behältnis (11), das ein Volumen abgrenzt, in dem die Ohrmatrix (1) untergebracht ist.

5. Verfahren zur Realisierung einer Hörvorrichtung nach Anspruch 4, **dadurch gekennzeichnet, dass** das Behältnis (11) mit einem halbflüssigen Werkstoff mit Formgedächtnis, wie zum Beispiel ein Hydrocolloidgel, gefüllt ist; wobei der Werkstoff die Gegenform (10) bildet, die für die Herstellung des Ohrpassstückes (12) notwendig ist.

6. Verfahren zur Realisierung einer Hörvorrichtung nach einem der vorhergehenden Ansprüche, **dadurch gekennzeichnet, dass** der Schritt der Herstellung des Ohrpassstückes (12) einen Schritt des Einfügens eines Etiketts ohne Kontakt (13) zum Lesen/Schreiben von Informationen, wie zum Beispiel des Typs RFID, in der Gegenform (10), umfasst.

7. Vorrichtung für die Umsetzung des Verfahrens nach einem der Ansprüche 1 bis 6, **dadurch gekennzeichnet, dass** sie einen eher flachen Träger (2) umfasst, der einen Vorratsbehälter (3), der sich zurückgesetzt zur Ebene (5) des Trägers (2) erstreckt, sowie einen mobilen Kolben (4) im Vorratsbehälter (3) aufweist.

8. Vorrichtung nach Anspruch 7, **dadurch gekennzeichnet, dass** der Vorratsbehälter (3) aus einem Zylinderrohr besteht.

9. Individuelle Vorrichtung zum Hörschutz, **dadurch gekennzeichnet, dass** sie ein Ohrpassstück (12) für jedes Ohr des Benutzers umfasst, wobei die Ohrpassstücke (12) anhand des Verfahrens nach den Ansprüchen 1 bis 6 realisiert und hergestellt werden.

10. Vorrichtung nach Anspruch 9, **dadurch gekennzeichnet, dass** die Ohrpassstücke (12) zumindest ein Etikett ohne Kontakt (13) zum Lesen/Schreiben von Informationen, wie zum Beispiel des Typs RFID, umfassen.

11. Vorrichtung nach einem der Ansprüche 9 bis 10, **dadurch gekennzeichnet, dass** sie eine Griff-Aufnahme-Einheit umfasst, wobei jedes der Ohrpassstücke (12) am Aufnahmeelement (16) befestigt ist.

12. Vorrichtung nach Anspruch 11, **dadurch gekennzeichnet, dass** das Griffelement (17) eine Lagerung (19) zum Lagern eines Metallteils (22) umfasst, um seine Erkennung beispielsweise durch Variierung eines Magnetfeldes sicher zu stellen.

13. Vorrichtung nach Anspruch 11, **dadurch gekennzeichnet, dass** das Aufnahmeelement (16) einen Schiebeteil (18) in einem zusätzlichen Formteil des Griffelements (17) umfasst; wobei die beiden Teile beispielsweise Zylinderformen aufweisen.

14. Vorrichtung nach Anspruch 13, **dadurch gekennzeichnet, dass** der Griff (17) zumindest zwei Membranen umfasst, die sich in seinem Teil gegenüber dem zusätzlichen Schiebeteil (18) des Aufnahmeelements (16) befinden, um gegenüber liegende Flächen zu bilden.

15. Vorrichtung nach Anspruch 11, **dadurch gekennzeichnet, dass** die beiden Ohrpassstücke (12) durch ein Kabel (21) miteinander verbunden sind, das beispielsweise am Griffelement (17) befestigt ist.

16. Vorrichtung nach Anspruch 11, **dadurch gekennzeichnet, dass** die beiden Ohrpassstücke (12) durch einen biegsamen Bügel (26) miteinander verbunden sind, deren jeweilige Befestigung, die beispielsweise durch Verschrauben auf dem jeweiligen Griffelement (17) sicher gestellt wird, an den Passstücken (12) derart einstellbar ist, dass ihnen ein variabler Druck F verliehen wird.

17. Vorrichtung nach einem der Ansprüche 9 bis 16, **dadurch gekennzeichnet, dass** jedes Ohrpassstück (12) ein Erkennungszeichen, wie zum Beispiel eine andere Farbe, aufweist, das es erlaubt, das Passstück des linken Ohres (12) und das Passstück des rechten Ohres zu identifizieren.

18. Vorrichtung nach Anspruch 11, **dadurch gekennzeichnet, dass** die Griff-Aufnahme-Einheit aus Kunststoff ist, oder aus jedem anderen Material, das anders als das der Ohrpassstücke (12) ist.

19. Vorrichtung nach Anspruch 14, **dadurch gekennzeichnet, dass** das Volumen zwischen den beiden Membranen zwischen 0,14 cm³ und 0,006 cm³ beträgt.
